(19) Europäisches Patentamt

European Patent Office

Office européen des brevets

(11) Veröffentlichungsnummer: **0 300 379**
**A2**

(12) EUROPÄISCHE PATENTANMELDUNG

(21) Anmeldenummer: 88111392.2

(22) Anmeldetag: 15.07.88

(51) Int. Cl.⁴: **A61K 7/06 , A61K 7/50 , A61K 7/08**

(30) Priorität: 24.07.87 DE 3724547

(43) Veröffentlichungstag der Anmeldung:
25.01.89 Patentblatt 89/04

(84) Benannte Vertragsstaaten:
AT BE CH DE ES FR GB GR IT LI NL SE

(71) Anmelder: Henkel Kommanditgesellschaft auf
Aktien
Postfach 1100 Henkelstrasse 67
D-4000 Düsseldorf-Holthausen(DE)

(72) Erfinder: Scheuffgen, Ingeborg
Spulgasse 3
D-4040 Neuss(DE)

(54) Alkanolamidfreies Perlglanzkonzentrat.

(57) Perlglanzkonzentrate in Form fließfähiger, wäßriger Dispersionen, die frei von Fettsäurealkanolamiden sind, enthalten

(A) 5 bis 20 Gew.-% lineare gesättigte Fettsäuren mit 16 bis 22 C-Atomen;

(B) 3 bis 10 Gew.-% eines oder mehrerer Emulgatoren.

Bevorzugt sind zusätzlich 1 bis 6 Gew.-% Ester der Formel $R^1-(OC_nH_{2n})_x-OR^2$ enthalten, worin $R^1$ eine Fettacylgruppe mit 16 bis 22 C-Atomen und $R^2$ Wasserstoff oder eine Gruppe $R^1$ ist. Als Emulgatoren sind nichtionogene Tenside bevorzugt.

EP 0 300 379 A2

## Alkanolamidfreies Perlglanzkonzentrat

Gegenstand der Erfindung ist ein Perlglanzkonzentrat in Form einer fließ- oder pumpfähigen, wäßrigen Dispersion einer linearen, gesättigten Fettsäure mit 16 bis 22 C-Atomen.

Wäßrigen Zubereitungen von Tensiden und kosmetischen Präparaten kann man durch Einarbeitung von Substanzen, die nach dem Abkühlen in Form feiner, perlmuttartig aussehender Kristalle ausfallen und in den Zubereitungen dispergiert bleiben, ein perlglänzendes, ästhetisch ansprechendes Aussehen verleihen. Als perlglanzbildende Stoffe eignen sich z.B. die Mono- und Diester aus Ethylenglycol, Propylenglycol und oligomeren Alkylenglycolen dieser Art oder Glycerin mit $C_{16}$-$C_{22}$-Fettsäuren sowie Monoalkanolamide von $C_{12}$-$C_{22}$-Fettsäuren mit Alkanolaminen mit 2 oder 3 C-Atomen.

Es ist auch bekannt, die genannten Perlglanzbildner in Wasser oder in wäßrigen Tensidlösungen stabil zu dispergieren und die auf diese Weise erhaltenen konzentrierten Perlglanzdispersionen den perlglänzend auszustattenden Zubereitungen ohne Erwärmung zuzusetzen, so daß sich das für die Einarbeitung sonst erforderliche Erwärmen und Abkühlen zur Bildung der Perlglanzkristalle erübrigt.

Perlglanzkonzentrate auf Basis der genannten Perlglanzbildner sind z.B. aus DE-A-16 69 152, aus JP-56/71021 (Chem. Abstr. 95/156360), aus DE-A-34 11 328 und DE-A-35 19 081 bekannt.

Die aus diesen Druckschriften bekannten Konzentrate enthalten als Teil der perlglanzbildenden Stoffe Fettsäure-mono- oder dialkanolamide. Alkanolamine und deren Derivate werden aber in jüngster Zeit verdächtigt, an der Bildung von Nitrosaminen beteiligt zu sein, und es besteht daher die Bestrebung, bei der Formulierung kosmetischer Zubereitungen ohne solche Alkanolamine und Alkanolamin-Derivate auszukommen. Ein Weglassen der Fettsäurealkanolamide aus den bekannten Perlglanz-Konzentraten führt zu einer nicht tragbaren Verringerung der perlglänzenden Eigenschaften. Es bestand daher die Aufgabe, ein Perlglanzkonzentrat in Form einer fließfähigen oder pumpfähigen Dispersion zu finden, welches von Alkanolamiden frei ist und einen über viele Monate lagerstabilen Perlglanz aufweist und geeignet ist, bei Zusatz zu wäßrigen Zubereitungen wasserlöslicher oberflächenaktiver Stoffe diesen ein perlglänzendes Aussehen zu verleihen. Diese Aufgabe wurde erfindungsgemäß gelöst durch ein Perlglanzkonzentrat in Form einer fließfähigen, wäßrigen Dispersion, die gekennzeichnet ist durch einen Gehalt von

(A) 5 bis 20 Gew.-% einer linearen, gesättigten Fettsäure mit 16 bis 22 C-Atomen und
(B) 3 bis 10 Gew.-% eines oder mehrerer Emulgatoren.

Einen besonders brillanten Perlglanz weisen erfindungsgemäße Perlglanzkonzentrate auf, wenn sie zusätzlich

(C) 1 bis 6 Gew.-% eines oder mehrerer Ester der allgemeinen Formel $R^1$-$(OC_nH_{2n})_x$-$OR^2$, in der $R^1$ eine lineare Fettacylgruppe mit 16 bis 22 C-Atomen, $R^2$ Wasserstoff oder eine Acylgruppe $R^1$, n = 2 oder 3 und x eine Zahl von 1 bis 4 ist.

Daneben enthalten die erfindungsgemäßen Perlglanzkonzentrate im wesentlichen Wasser in einer Menge von ca. 70 bis 90 Gew.-%. Zur Konservierung gegen Bakterien- und Pilzbefall werden handelsübliche Konservierungsmittel in untergeordneten Mengen zugegeben. Weiterhin können in untergeordneten Mengen Puffersubstanzen zur Einstellung des pH-Wertes auf Werte zwischen 6 und 8, z. B. Citronensäure und/oder Natriumcitrat enthalten sein.

Als lineare Fettsäuren (A) können z. B. Palmitinsäure, Stearinsäure, Arachinsäure oder Behensäure eingesetzt werden, geeignet sind aber auch technische Fettsäureschnitte, die ganz oder überwiegend aus Fettsäuren mit 16 bis 22 C-Atomen bestehen, z.B. Palmitin-Stearinsäure-Fraktionen wie sie aus Talgfettsäure durch Abtrennung der bei +5 °C flüssigen Fettsäuren gewonnen werden oder Palmitin-Stearinsäure-Fraktionen wie sie durch Härten von Talgfettsäure erhältlich sind.

Als Emulgatoren (B) können grenzflächenaktive Verbindungen unterschiedlicher Struktur oder Ionogenität verwendet werden, die sich durch eine lipophile, bevorzugt lineare, Alkyl- oder Alkenylgruppe und eine, bevorzugt endständig daran gebundene, hydrophile Gruppe auszeichnen. Die hydrophile Gruppe kann zum Beispiel eine ionogene Gruppe, z.B. eine Sulfat- oder Carboxylat-Gruppe oder eine quartäre Ammoniumgruppe sein. Bevorzugt ist als hydrophile Gruppe jedoch eine nichtionogene Gruppe, z.B. eine Polyolgruppe, eine Polyalkylenglycolethergruppe oder eine Kombination aus Polyol- und Polyglycolethergruppe. Obwohl also prinzipiell alle anionischen, zwitterionischen, ampholytischen und kationischen Tenside für die Herstellung der erfindungsgemäßen Perlglanzdispersion geeignet sind, stellen Perlglanzdispersionen, die einen nichtionogenen Emulgator (B) enthalten, eine bevorzugte Ausführungsform der Erfindung dar, da sie mit kationischen wie mit anionischen Tensidzubereitungen gleichermaßen verträglich sind. Besonders bevorzugt sind solche Perlglanzkonzentrate, die als Emulgatoren nichtionogene Tenside aus der Gruppe der

(B1) Anlagerungsprodukte von 2 bis 30 Mol Ethylenoxid und 0 bis 5 Mol Propylenoxid an Fettalkohole mit 8 bis 18 C-Atomen, an Fettsäuren mit 12 bis 18 C-Atomen, an Alkylphenole mit 8 bis 15 C-Atomen in der Alkylgruppe,

(B2) $C_{12}$-$C_{18}$-Fettsäuremono- und -diester von Anlagerungsprodukten von 1 bis 30 Mol Ethlyenoxid an Glyerin,

(B3) Glycerinmono- und -diester und Sorbitanmono- und -diester von gesättigten und ungesättigten $C_8$-$C_{18}$-Fettsäuren und deren Ethylenoxidanlagerungsprodukte,

(B4) $C_8$-$C_{18}$-Alkylmono- und -oligoglycoside oder

(B5) Anlagerungsprodukte von 10 bis 60 Mol Ethylenoxid an Ricinusöl und gehärtetes Ricinusöl enthalten.

Die Anlagerungsprodukte von Ethylenoxid und ggf. von Propylenoxid an Fettalkohole, Fettsäuren, Alkylphenole, Glycerin-mono- und -diester sowie Sorbitanmono- und -diester von Fettsäuren oder an Ricinusöl stellen bekannte, im Handel erhätliche Produkte dar. Es handelt sich dabei um Homologengemische, deren mittlerer Alkoxylierungsgrad der angelagerten molaren Menge an Ethylenoxid bzw. Propylenoxid entspricht.

$C_{12}$-$C_{18}$-Fettsäuremono- und -diester von Anlagerungsprodukten von Ethylenoxid an Glycerin sind aus DE-PS 20 24 051 als Rückfettungsmittel für kosmetische Zubereitungen bekannt. $C_8$-$C_{18}$-Alkylmono- und oligoglycoside, ihre Herstellung und ihre Verwendung als oberflächenaktive Stoffe sind beispielsweise aus US-A 3,839,318, US-A 3,707,535, US-A 3,547,828, DE-A 19 43 689, DE-A 20 36 472 und DE-A 30 01 064 sowie EP-A 77 167 bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise 8 geeignet sind. Der Oligomerisierungsgrad ist dabei ein statistischer Mittelwert, dem eine für solche technischen Produkte übliche Homologenverteilung zugrunde liegt.

Neben den genannten, bevorzugt enthaltenen nichtionogenen Emulgatoren können als anionische Emulgatoren z.B. Alkylsulfate mit 12 bis 18 C-Atomen in der Alkylgruppe, Alkylpolyethylenglycolethersulfate mit 12 bis 16 C-Atomen in der Alkylgruppe und 1 bis 6 Ethylenglycolethergruppen im Molekül, in Form ihrer Alkali-, Magnesium-, Ammonium-, Mono-, Di- oder Trialkanolammoniumsalze mit 2 bis 3 C-Atomen in der Alkanolgruppe eingesetzt werden. Weitere geeignete Aniontenside sind Alkansulfonate, Alpha-Olefinsulfonate, Alpha-Sulfofettsäuremethylester, Sulfobernsteinsäureester-Salze, Acylisethionate und Acylsarcoside. Auch Seifen können als Emulgatoren verwendet werden. Dies kann z. B. dadurch erreicht werden, daß man einen kleinen Teil, etwa 1 bis 20 Gew.-% der linearen, gesättigten Fettsäuren durch zugesetztes Alkalihydroxid verseift und auf diese Weise in einen anionischen Emulgator überführt.

Als kationische Emulgatoren können quartäre Ammoniumtenside, z.B. Cetyltrimethylammoniumchlorid, Cetylpyridiniumchlorid, Stearyltrimethylammoniumchlorid, Lauryldimethylbenzylammoniumchlorid oder Talgalkyltris-(oligooxyalkyl)-ammonium-phosphat eingesetzt werden. Schließlich eignen sich auch zwitterionische Tenside wie z. B. das Kokosalkyl-dimethylammonium-glycinat, das Kokosacylaminopropyl-dimethylammoniumglycinat oder das Kokosacylaminoethyl-hydroxyethyl-carboxymethyl-glycinat oder ampholytische Tenside wie z.B. das $C_{12-18}$-Acylsarcosin, das N-Kokosalkylaminopropionat oder das Kokosacylaminoethylaminopropionat.

Ionogene Tenside werden bevorzugt gemeinsam mit nichtionogenen Tensiden eingesetzt. Erfindungsgemäße Perglanzkonzentrate, die keine ionischen Tenside enthalten, haben sich jedoch als besonders universell einsetzbar und mit wäßrigen Zubereitungen wasserlöslicher oberflächenaktiver Stoffe beliebiger Art und Ionogenität als besonders gut verträglich erwiesen.

Als Ester (C) der allgemeinen Formel $R^1(OC_nH_{2n})_xOR^2$ können z.B. die Mono- und Diester des Ethylenglycols und Propylenglycols mit höheren Fettsäuren, z.B. mit Palmitinsäure, Stearinsäure oder Behensäure oder die Diester des Diethylenglycols oder des Triethylenglycols mit solchen Fettsäuren eingesetzt werden. Geeignet sind auch Mischungen von Mono- und Diestern der genannten Glycole mit Fettsäuregemischen, z.B. mit gehärteter Talgfettsäure oder mit der gesättigten $C_{16}$-$C_{18}$-Fettsäurefraktion der Talgfettsäure. Bevorzugt geeignet ist der Ethylenglycolmono- und/oder Diester der Palmitin- und/oder Stearinsäure.

Handelsübliche Ethylenglycolstearate enthalten z.B. 50 bis 90 Gew.-% Distearat und 10 bis 50 Gew.-% Monostearat.

In einer bevorzugten Ausführungsform enthält das erfindungsgemäße Perlglanzkonzentrat

(A) 10 bis 20 Gew.-% Palmitin- und/oder Stearinsäure,

(B) 3 bis 10 Gew.-% eines Emulgators aus der Gruppe der

(B1) Anlagerungsprodukte von 6 bis 16 Mol Ethylenoxid an Fettalkohole mit 12 bis 18 C-Atomen und/oder der

(B2) $C_{12}$-$C_{18}$-Fettsäure-mono- und -diester von Anlagerungsprodukten von 5 bis 15 Mol Ethylenoxid an Glycerin.

In einer besonders bevorzugten Ausführung enthält das erfindungsgemäße Perlglanzkonzentrat zusätzlich

(C) 1 bis 6 Gew.-% Ethylenglycoldiester von Palmitin- und/oder Stearinsäure.

Besonders stabile, brillanten Perlglanz verleihende sowie gut fließ- oder pumpfähige Zusammensetzungen enthalten

ca. 15 bis 20 Gew.-% der Komponente (A),

ca. 3 bis 5 Gew.-% der Komponente (B1),

ca. 0 bis 5 Gew.-% der Komponente (B2) und

ca. 3 bis 5 Gew.-% der Komponente (C).

Die Herstellung der erfindungsgemäßen Perlglanzkonzentrate erfolgt bevorzugt in der Weise, daß die Komponenten (A), (B) und (C) zunächst gemeinsam über ihren Schmelzpunkt, bevorzugt auf eine Temperatur von 60 bis 70 °C erwärmt und vermischt werden. Wird als Emulgator ein ionisches, wasserlösliches Tensid eingesetzt, so empfiehlt es sich, dieses in der Wasserphase aufzulösen. Die wäßrige Phase kann auch bereits gegebenenfalls die Puffersubstanzen gelöst enthalten. Soll ein Teil der Fettsäure in die Seife überführt werden, so wird die zur Verseifung erforderliche Menge Kaliumhydroxid oder Natriumhydroxid ebenfalls in der Wasserphase aufgelöst. In die auf 60 bis 80 °C erwärmte Wasserphase wird sodann unter Rühren die Schmelze der Komponenten (A), (B) und (C) eingemischt. Die entstehende Emulsion wird dann innerhalb von wenigen Minuten auf 50 bis 60 °C abgekühlt und bei dieser Temperatur mit einem Homogenisator oder Dispergieraggregat, welches hohe Scherkräfte entwickelt, für wenige Minuten homogenisiert. Hierfür eignen sich statische und dynamische Mischaggregate, z. B. Spalthomogenisatoren oder Dispergiergeräte, die nach dem Stator-Rotor-Prinzip arbeiten. Nach dieser kurzen und intensiven Homogenisierung wird die dabei gebildete Dispersion unter langsamem Rühren weiter auf Raumtemperatur abgekühlt. Temperaturempfindliche Konservierungsmittel sollten erst nach Abkühlung unterhalb +40 °C zugesetzt werden.

Die erfindungsgemäßen Perlglanzkonzentrate eignen sich zur Herstellung getrübter und perlglänzender flüssiger, wäßriger Zubereitungen wasserlöslicher oberflächenaktiver Stoffe. Zur Erzeugung von Perlglanz werden den klaren wäßrigen Zubereitungen bei 0 bis 40 °C erfindungsgemäße Perlglanzkonzentrate in einer Menge von 1 bis 10 Gew.-% der Zubereitung zugesetzt und unter Rühren darin verteilt. Obwohl in vielen Fällen erfindungsgemäße Perlglanzkonzentrate mit kationischen oder anionischen Emulgatoren auch mit Tensidzubereitungen entgegengesetzter Ionogenität verträglich sind, empfiehlt es sich, zur Herstellung von getrübten oder perlglänzenden wäßrigen Zubereitungen ionogener Tenside solche erfindungsgemäßen Perlglanzkonzentrate zu verwenden, die einen Emulgator gleicher Ionogenität oder einen nichtionogenen Emulgator enthalten.

Die folgenden Beispiele sollen den Erfindungsgegenstand näher erläutern:

## Beispiele

Die in Tabelle I aufgeführten Perlglanzkonzentrate wurden wie folgt hergestellt:

Die Fettkomponenten (Palmitin-/Stearinsäure, Ethylenglycolmono-/distearat und Emulgatoren) wurden zusammengegeben, über den Schmelzpunkt erhitzt und bei ca. 65 °C gemischt. Das Konservierungsmittel (P-Hydroxybenzoesäureester-Gemisch, PHENONIP(R)) und in den Beispielen 1 bis 15 und 22 bis 23 das Kaliumhydroxid wurden in Wasser gelöst und die Lösung auf ca. 70 °C erhitzt. Unter Rühren wurde dann die Schmelze der Fettkomponenten in die Wasserphase eingemischt.

Nach ca. 15 Minuten Rühren wurde die Dispersion rasch auf 56 °C unter weiterem Rühren abgekühlt, dann langsam bis 50 °C gekühlt und mit einem Spalthomogenisator 4 Minuten lang homogenisiert. Danach wurde unter langsamem Rühren auf Raumtemperatur (ca. 25 °C) abgekühlt.

## Tabelle I   (Perlglanzkonzentrate)

| Beispiel Nr. | 1 | 2-3 | 4-12 | 13 | 14 | 15 | 16 | 17 | 18 | 19 | 20+21 | 22+23 |
|---|---|---|---|---|---|---|---|---|---|---|---|---|
| Palmitin-/Stearin-säure (50:50) | 12 | 12 | 12 | 15 | 15 | 15 | 14 | 14 | 12 | 15 | 20 | 12 |
| Ethylenglycolmono/distearat (1:2) | – | 3 | 3 | – | – | 4 | 4 | 4 | 3 | 4 | 4 | 3 |
| KOH (25 Gew.-%ige Lösung in Wasser) | 0,5 | 0,2 | 0,25 | 0,5 | 0,5 | 0,25 | – | – | – | – | – | 0,025 |
| Emulgator | 6 | 9 | 8 | 4 | 6 | 3 | 3 | 7 | 8 | 3 | 7 | 6 |
| P-Hydroxybenzoesäu-reester (Phenonip$^{(R)}$) | 0,2 | 0,2 | 0,2 | 0,2 | 0,2 | 0,2 | 0,2 | 0,2 | 0,2 | 0,2 | 0,2 | 0,2 |
| Wasser | ad 100 | ad 100 | ad 100 | ad 100 | ad 100 | ad 100 | ad 100 | ad 100 | ad 100 | ad 100 | ad 100 | ad 100 |

EP 0 300 379 A2

Der in den Beispielen 1 bis 22 verwendete Emulgator hatte die folgende Zusammensetzung:

Beispiel 1:
4 Gew.-Teile Sorbitanmonolaurat
2 Gew.-Teile Sojaölfettsäuremonoglycerid

Beispiel 2:
4 Gew.-Teile Sorbitanmonolaurat
2 Gew.-Teile Kokosfettalkohol($C_{12-13}$) + 10 Mol EO
3 Gew.-Teile Glycerin + 7 Mol EO-Kokosfettsäure($C_{12-13}$)-monoester

Beispiel 3:
5 Gew.-Teile Kokosfettalkohol($C_{12-14}$) + 3 Mol EO
4 Gew.-Teile Sorbitanmonolaurat

Beispiel 4:
3 Gew.-Teile Glycerin-monostearat + 24 Mol EO
2 Gew.-Teile Kokosfettalkohol($C_{12-13}$) + 10 Mol EO
3 Gew.-Teile Sorbitanmonolaurat

Beispiel 5:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Kokosfettalkohol($C_{12-14}$) + 4,5 Mol EO + 5 Mol PO

Beispiel 6:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Sorbitanmonolaurat

Beispiel 7:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Sorbitanmonostearat

Beispiel 8:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Sorbitanmonooleat

Beispiel 9:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Alkyl($C_{8-10}$)-oligo(1,3)-glucosid

Beispiel 10:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Ricinusöl + 40 Mol EO

Beispiel 11:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Glycerinmono-/dilaurat + 20 Mol EO

Beispiel 12:
3 Gew.-Teile Glycerin + 7 Mol EO-Kokosfettsäure($C_{12-13}$)-monoester
2 Gew.-Teile Kokosfettalkohol($C_{12-13}$) + 10 Mol EO
3 Gew.-Teile Glycerinmonooleat + 25 Mol EO

Beispiel 13:
Sorbitanmonolaurat

Beispiel 14:
4 Gew.-Teile Sorbitanmonolaurat
2 Gew.-Teile 2-Ethylhexansäure-monoglycerid

Beispiel 15:
1 Gew.-Teile Cetyl-Stearylalkohol(1:1) + 12 Mol EO
2 Gew.-Teile Kokosfettalkohol($C_{12-13}$) + 10 Mol EO

Beispiel 16:
2 Gew.-Teile Hydriertes Ricinusöl + 40 Mol EO

6

1 Gew.-Teile Cetyl-Stearylalkohol(1:1) + 12 Mol EO

Beispiel 17:

2 Gew.-Teile Kokosfettalkohol($C_{12-18}$) + 10 Mol EO

2 Gew.-Teile Cetyl-Oleylalkohol + 5 Mol EO

3 Gew.-Teile Sorbitanmonolaurat

Beispiel 18:

3 Gew.-Teile Glycerin + 7 Mol EO-Kokosfettsäure($C_{12-18}$)-monoester

2 Gew.-Teile Kokosfettalkohol($C_{12-18}$) + 10 Mol EO

3 Gew.-Teile Cetyl-Stearylalkohol(30:70) + 5 Mol EO

Beispiel 19:

2 Gew.-Teile Kokosfettalkohol($C_{12-18}$) + 10 Mol EO

1 Gew.-Teile Ethyl-/Stearylalkohol(1:1) + 12 Mol EO

Beispiel 20:

2 Gew.-Teile Kokosfettalkohol($C_{12-18}$) + 10 Mol EO

2 Gew.-Teile Cetyl-/Stearylalkohol(1:1) + 12 Mol EO

3 Gew.-Teile Glycerin + 7 Mol EO-Kokosfettsäure($C_{12-18}$)-monoester

Beispiel 21:

2 Gew.-Teile Cetyltrimethylammoniumchlorid (30 Gew.-%ige wäßrige Lösung)

2 Gew.-Teile Cetyl-Stearylalkohol(1:1) + 12 Mol EO

3 Gew.-Teile Glycerin + 7 Mol EO-Kokosfettsäure($C_{12-18}$)-monoester

Beispiel 22:

3 Gew.-Teile Glycerin + 7 Mol EO-Kokosfettsäure($C_{12-18}$)-monoester

2 Gew.-Teile Kokosfettalkohol($C_{12-18}$) + 10 Mol EO

1 Gew.-Teil Cetyl-/Stearyl(1:1)-sulfat, Na-Salz

Beispiel 23:

3 Gew.-Teile Glycerin + 7 Mol EO-Kokosfettsäure($C_{12-18}$)-monoester

2 Gew.-Teile Kokosfettalkohol($C_{12-18}$) + 10 Mol EO

1 Gew.-Teil Laurylsulfat, Na-Salz

## Anwendungsbeispiele

### 24: Anionisches Shampoo

Fettalkohol($C_{12-14}$)-polyglycol(2 EO)ether-sulfat, Na-Salz, 28%ig in Wasser    38 g

N-Hydroxyethyl-N-Kokosacylaminoethyl-(carboxymethyl)-glycinat,  Na-Salz  (CTFA:  Cocoamphoglycinate), 30%ig in Wasser    10 g

Glycerin + 7 Mol EO-Kokosfettsäure-($C_{12-18}$)-monoester    2 g

Natriumchlorid    1,0 g

Perlglanzkonzentrat nach Beispiel 16    8,0 g

Wasser    41 g

### 25: Ampholytisches Shampoo

N-Hydroxyethyl-N-Kokosacylaminoethyl-aminopropionat (CTFA: Cocoamphodipropionate), 40%ig in Wasser    41,0 g

PEG 120 Methylglucose-dioleat (Glucamate[R]DOE 120, AMERCHOL)    5,0 g

Perlglanzkonzentrat nach Beispiel 20    9 g

Wasser    45 g

26: Schaumbad/Duschbad

Ammoniumlauryl($C_{12-15}$)sulfat, 30%ig in Wasser      40 g
Kokosacylaminopropyl-dimethylammoniumglycinat (CTFA: cocoamidopropylbetaine), 30%ig in Wasser
15 g
Perlglanzkonzentrat nach Beispiel 19      8 g
Wasser      37 g


27: Haarnachbehandlungsmittel

Tris(oligooxyethyl)-alkyl($C_{16/18}$)ammoniumphosphat, 50%ig in Wasser (CTFA: Quaternium-52)      2,0 g
Glycerin + 7 Mol-Kokosfettsäure($C_{12-18}$)-monoester      0,5 g
Hydroxyethylcellulose (2%ig in Wasser)      50,0 g
Perlglanzkonzentrat nach Beispiel 18      3,0 g
Zitronensäure      0,2 g
Wasser      64,3 g


28: Schnellhaarkur

Polyglycol-Polyamin-Kondensationsharz (CTFA: PEG-15-Tallow-Polyamine)      5,0 g
Hydroxyethylcellulose (2%ig in Wasser)      92,0 g
Perlglanzkonzentrat nach Beispiel 17      3,0 g


## Ansprüche

1. Perlglanzkonzentrat in Form einer fließfähigen, wäßrigen Dispersion, dadurch gekennzeichnet, daß es
(A) 5 bis 20 Gew.-% einer linearen, gesättigten Fettsäure mit 16 bis 22 C-Atomen und
(B) 3 bis 10 Gew.-% eines oder mehrerer Emulgatoren
enthält.
2. Perlglanzkonzentrat nach Anspruch 1, dadurch gekennzeichnet, daß zusätzlich
(C) 1 bis 6 Gew.-% eines oder mehrerer Ester der allgemeinen Formel $R^1$-$(OC_nH_{2n})_x$-$OR^2$, in der $R^1$ eine lineare Fettacylgruppe mit 16 bis 22 C-Atomen, $R^2$ Wasserstoff oder eine Acylgruppe $R^1$, n = 2 oder 3 und x eine Zahl von 1 bis 4 ist,
enthalten sind.
3. Perlglanzkonzentrat nach den Ansprüchen 1 oder 2, dadurch gekennzeichnet, daß als Emulgatoren nichtionogene Tenside aus der Gruppe der
(B1) Anlagerungsprodukte von 2 bis 30 Mol Ethylenoxid und 0 bis 5 Mol Propylenoxid an lineare Fettalkohole mit 8 bis 18 C-Atomen, an Fettsäuren mit 12 bis 18 C-Atomen, an Alkylphenole mit 8 bis 15 C-Atomen in der Alkylgruppe,
(B2) $C_{12}$-$C_{18}$-Fettsäuremono- und -diester von Anlagerungsprodukten von 1 bis 30 Mol Ethlyenoxid an Glyerin,
(B3) Glycerinmono- und -diester und Sorbitanmono- und -diester von gesättigten und ungesättigten $C_8$-$C_{18}$-Fettsäuren und deren Ethylenoxidanlagerungsprodukte,
(B4) $C_8$-$C_{18}$-Alkylmono- und -oligoglycoside oder
(B5) Anlagerungsprodukte von 10 bis 60 Mol Ethylenoxid an Ricinusöl und gehärtetes Ricinusöl
enthalten sind.
4. Perlglanzkonzentrat nach den Ansprüchen 1 bis 3, dadurch gekennzeichnet, daß
(A) 10 bis 20 Gew.-% Palmitin- und/oder Stearinsäure
(B) 3 bis 10 Gew.-% eines Emulgators aus der Gruppe der
(B1) Anlagerungsprodukte von 6 bis 16 Mol Ethylenoxid an Fettalkohole mit 12 bis 18 C-Atomen und/oder der

8

(B2) $C_{12}$-$C_{18}$-Fettsäure-mono- und -diester von Anlagerungsprodukten von 5 bis 15 Mol Ethylenoxid an Glycerin

enthalten sind.

5. Perlglanzkonzentrat nach den Ansprüchen 1 bis 4, dadurch gekennzeichnet, daß zusätzlich

(C) 1 bis 6 Gew.-% Ethylenglycoldiester von Palmitin- und/oder Stearinsäure

enthalten ist.

6. Perlglanzkonzentrat nach den Ansprüchen 2 bis 5, dadurch gekennzeichnet, daß die Komponenten (A) und (C) in einem Gewichtsverhältnis von 70 : 30 bis 80 : 20 und zusammen in einer Menge von 15 bis 20 Gew.-% enthalten sind.

7. Verfahren zur Herstellung getrübter und perlglänzender flüssiger, wäßriger Zubereitungen wasserlöslicher oberflächenaktiver Stoffe, dadurch gekennzeichnet, daß man den klaren wäßrigen Zubereitungen bei 0 bis 40 °C Perlglanzkonzentrate gemäß Anspruch 1 bis 6 in einer Menge von 1 bis 10 Gew.-% der Zubereitung zusetzt und unter Rühren darin verteilt.